# EUROPEAN PATENT APPLICATION

(11) **EP 4 516 261 A2**
(43) Date of publication of application: **05.03.2025**
(21) Application number: 23789361.5
(22) Date of filing: 25.04.2023
(51) Int. Cl.: A61C 1/08, A61B 34/20, A61C 9/00

(54) **DENTAL HANDPIECE**

(30) Priority: 25.04.2022 RU 2022111203
(71) Applicant: Mikhalskiy, Alexander, 1246 Corsier (CH)
(72) Inventor: LIPPGARDT, Ivan Georgievich, Moscow, 115404 (RU); SOLOVYKH, Evgenii Anatol'evich, Moscow, 125362 (RU); ZADUROV, Sergey Igorevich, Moscow, 117534 (RU); BLINKOV, Valentin Alekseevich, Moscow, 111401 (RU); TERKULOVA, Elena Vadimovna, Moscow, 127422 (RU); SOLOVYKH, Anastasiya Evgen'evna, Moscow, 105094 (RU); ABDULLIN, Ramil Ilfatovich, Moscow, 109443 (RU); ABAKUMOV, Nikolai Konstantinovich, Obninsk, 249034 (RU); KIM, Andrei Evgenevich, Kommunarka, 108814 (RU); TUMBAKOV, Aleksandr Petrovich, Samara, 443023 (RU)
(74) Representative: Koudine, Andreï
(86) International application number: PCT/IB2023/054260
(87) International publication number: WO 2023/209575

(57) **Abstract**

The invention relates to a dental handpiece comprising a handle and a housing with a first light-emitting diode and a first optical sensor. According to the invention, the housing comprises a second light-emitting diode, a second optical sensor, shielding windows for each optical sensors. The dental handpiece comprises a means for supplying compressed air on each shielding window. The housing comprises apertures for supplying compressed air to each shielding window. A diffuser is located in each aperture, said diffuser being adapted to generate an air jet forming an air curtain. The technical problem is to provide a reliable protection of the optical sensors by implementing of the air curtain and obtaining a high-quality three-dimensional image of an oral cavity during manipulation in it with said dental handpiece.

## Description

The present invention relates to a dentistry equipment, and more specifically, to dental handpieces comprising a handle and a housing with an optical emission source represented by at least one light-emitting diode connected to an electric power supply, said handpiece having an optical sensor adapted to be connected to visualization devices, said handpiece can be used by dentists for dental procedures.

A known example of prior art is a dental handpiece comprising a handle and a housing with an optical emission source represented by at least one light-emitting diode connected to an electric power supply, said housing comprising an optical sensor adapted to be connected to visualization devices, see description of the utility model RU114603U1.

Said device is the closest art versus the claimed invention and is considered as a prototype. Thus, the claimed invention shall be described in terms of differences from the prototype.

A drawback of said device is that it has a video camera (an optical sensor) which allows to project an image of an oral cavity to an external screen but cannot ensure that no particles from the oral cavity get on its surface during a dental procedure. Indeed, certain tools, such as drills, can produce solid fragments flying at high speeds that are capable of crossing over ambient air and getting on the video camera.

Another major drawback of the prototype is that it does not allow to obtain a high-quality three-dimensional image of the oral cavity wherein the dental handpiece operates.

The present invention mainly aims to offer a dental handpiece comprising a handle and a housing with an optical emission source represented by at least one first light-emitting diode connected to an electric power supply, said dental handpiece having a first optical sensor adapted to be connected to visualization devices, said dental handpiece being capable of ensuring high-quality stable three-dimensional image, which is a stated technical problem.

For this purpose
- the housing has a second light-emitting diode connected to an electric power supply, said housing having a second optical sensor adapted to be connected to the visualization devices,
- the housing has a first transparent shielding window of the first optical sensor and a second transparent shielding window of the second optical sensor,
- the handpiece has a means for supplying compressed air on the first and second transparent shielding windows,
- the housing has a first aperture for supplying compressed air to the first transparent shielding window and a second aperture for supplying compressed air to the second transparent shielding window, both apertures being connected to the device for supplying compressed air,
- a diffuser is located in each aperture, said diffuser being designed so as to generate an air jet which forms an air curtain in front of the shielding window.

In addition from simply blowing on the transparent shielding window of the optical sensor to protect it from fogging, these useful features make it possible to create a powerful air curtain to protect said shielding window from contamination with solid particles and liquid droplets produced during a preparation of the patient's living tissues, that is, to protect the shielding window from getting any particles on external optical components, in other words, to ensure reliable protection of the optical sensor due to implementation of the air curtain. The second optical sensor is also reliably protected from getting any particles on external optical components, which makes it possible to create an image of an oral cavity in stereo.

Furthermore, a virtual 3D model can be generated using pre-rendered CT images. Said virtual 3D model can be joined with CT images using optical sensors to view hidden dental cavities so as to get an understanding how a cutting tip is positioned relative to these cavities.

There is a possible optional embodiment of the invention in which the housing has a third light-emitting diode connected to an electric power supply, said housing having a third optical sensor adapted to be connected to the visualization devices.

These useful features make it possible to produce an embodiment of the dental handpiece in which three optical sensors create a better-quality three-dimensional image.

There is a possible optional embodiment of the invention in which the housing has a fourth light-emitting diode connected to an electric power supply, said housing having a fourth optical sensor adapted to be connected to the visualization devices.

These useful features make it possible to propose an optional form of the dental handpiece in which four optical sensors create an even better-quality three-dimensional image.

There is a possible optional embodiment of the invention in which each diffuser is located from a working end of the housing and is designed so as to direct the air jet away from the housing end.

These useful features make it possible to propose an optional form of the dental handpiece, wherein optical sensors are located farther from the housing end than the drill bit. Under such conditions, the air jet is directed tangentially to each shielding window away from the working end of the housing, that is, under operational conditions, away from a patient's mouth.

Also, there is a possible alternative embodiment of the invention in which each diffuser is located from the housing handle and is designed so as to direct the air jet away from the handle. Under these conditions, the housing has a protrusion located from the working end, said protrusion having a surface shape which turns the air jet to 120°-140°.

These useful features make it possible to propose an alternative optional form of the dental handpiece, wherein the drill bit is located farther from the housing end than the optical sensors. The air jet is directed tangentially onto the shielding window and towards the housing end, then gets rotated by the protrusion, that is, under operational conditions, away from a patient's mouth.

In addition, since the housing has the protrusion located from the working end, said protrusion having a surface shape which turns the air jet to 120°-140°, the formed jet consists of two echelons with differently directed flows. Such complex flow of the high-speed jets makes it possible to increase an efficiency of interception of flying particles by extending their range in velocities and densities. This additionally prevents contamination of the optics.

There is another possible embodiment of the invention in which each diffuser is oriented perpendicularly to the handle. Said diffuser is designed so as to direct the air jet sideways from the handle. Under these conditions, the housing has a protrusion located from the working end, said protrusion having a surface shape which turns the air jet to 120°-140°.

These useful features make it possible to propose an optional form of the dental handpiece, wherein the optical sensor is located laterally to the drill bit, and the air jet is directed from a central axis of the housing and is rotated similarly to the embodiment described above.

Finally, there is a possible embodiment of the invention in which each diffuser is designed so as to generate the air jet having an outflow speed ranging from 6 m/s to 50 m/s, said outflow speed forming an angle from 0° to 15° with a plane of the transparent shielding window.

These useful features make it possible to propose an alternative optional form of the diffuser with target specifications and ensuring a maximum protection effect for the optical sensor.

Other distinguishing features and advantages of the invention are readily apparent from the description below, which includes for illustration but is not limited to the following features, with reference to the figures attached, where:

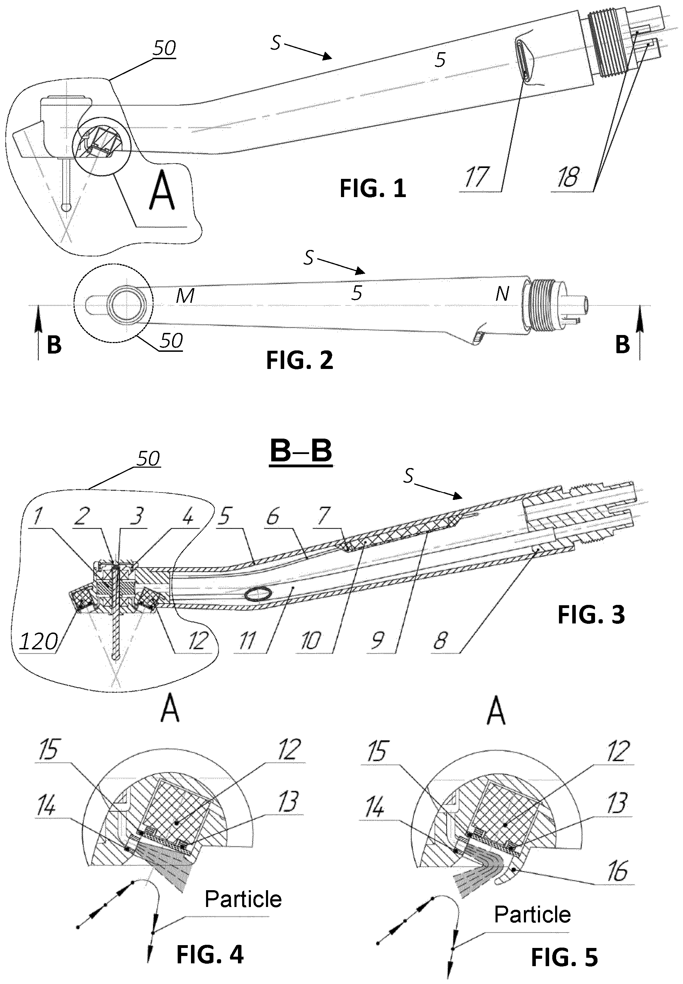

- figures 1 and 2 represent a design of a dental handpiece according to a first embodiment of the invention, wherein two optical sensors are located longitudinally (along an axis MN of a handle 5, figure 2) according to the invention; figure 1 represents a side view of the dental handpiece (with a partial cross-section A of housing 50 elements including the MN axis of the handle 5, figure 2), figure 2 represents a plan view of the dental handpiece;
- figure 3 represents a vertical longitudinal cross-section of the dental handpiece (along the MN axis of the handle 5, B-B plane, see figure 2) according to the first embodiment of the invention, having two optical sensors located longitudinally (along the MN axis of the handle 5, see figure 2);
- figures 4 and 5 represent an enlarged cross-section A of the of the housing 50 elements of the dental handpiece (see figure 1) that demonstrates a direct airjet and a reverse air jet blowing on a transparent shielding window, according to the invention;

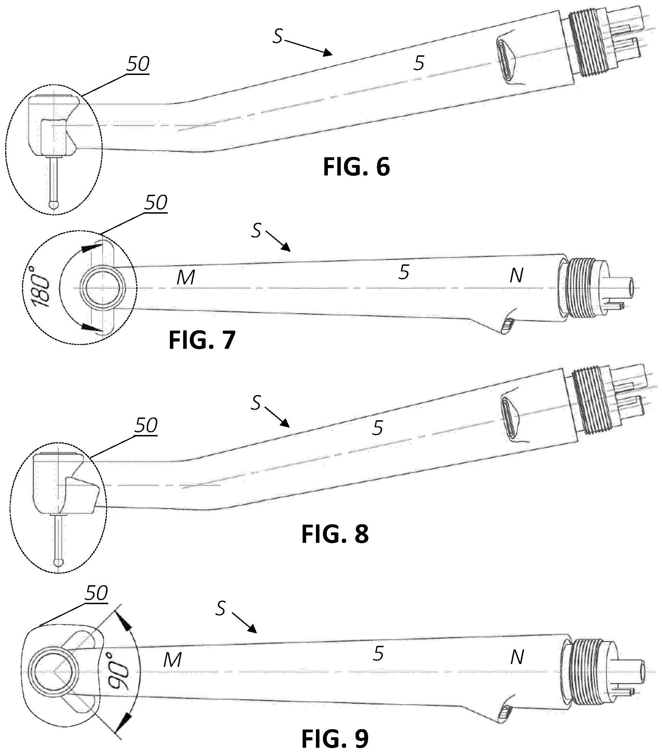

- figures 6 and 7 represent a dental handpiece according to a second embodiment of the invention, wherein two optical sensors are located transversally (across the axis MN of the handle 5, see figure 7); figure 6 represents a side view of the dental handpiece, figure 7 represents a plan view of the dental handpiece;
- figures 8 and 9 represent a dental handpiece according to a third embodiment of the invention, wherein two optical sensors are located at a 90° angle relative to each other (and at a 45° angle to the longitudinal axis MN of the handle 5, respectively, see figure 9); figure 8 represents a side view of the dental handpiece, figure 9 represents a plan view of the dental handpiece;

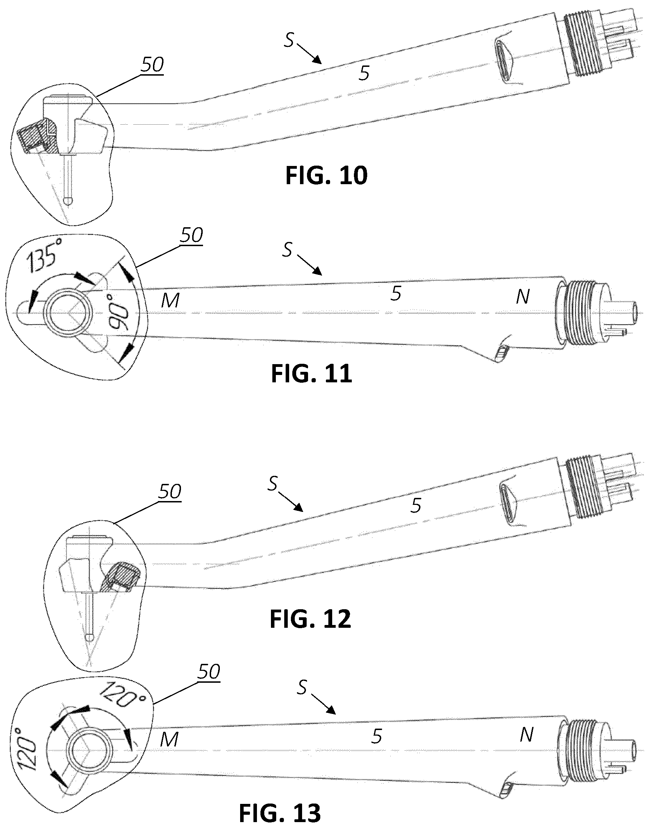

- figures 10 and 11 represent a cross-section of the dental handpiece according to a fourth embodiment of the invention with three optical sensors demonstrating a "direct" position, wherein a first optical sensor and a second optical sensor are located at a 90° angle relative to each other (and at a 45° angle to the longitudinal axis MN of the handle 5, respectively, see figure 11), and a third optical sensor is located along the longitudinal axis MN from the handle 5, an angle between the first optical sensor and the second optical sensor is equal to an angle between the second optical sensor and the third optical sensor and corresponds to 135° (figure 11); figure 10 represents a side view of the dental handpiece (with a partial cross-section of the housing 50 elements including the axis MN of the handle 5, figure 11), figure 11 represents a plan view of the dental handpiece;
- figures 12 and 13 represent a cross-section of the dental handpiece according to a fourth embodiment of the invention with three optical sensors demonstrating an "inverted" position, wherein a first optical sensor is located along the longitudinal axis MN, towards the handle 5 (figure 13), a second optical sensor is located on the one side (on the right in the figure 13) from the longitudinal axis MN, in direction from the handle 5, at a 120° angle relative to the longitudinal axis MN, a third optical sensor is located on the other side (on the left in the figure 13) from the longitudinal axis MN, in direction from the handle 5, at a 120° angle relative to the longitudinal axis MN, with an angle between the second optical sensor and the third optical sensor is also equal 120°; figure 12 represents a side view of the dental handpiece (with a partial cross-section of the housing 50 elements including the axis MN of the handle 5, figure 13), figure 13 represents a plan view of the dental handpiece;

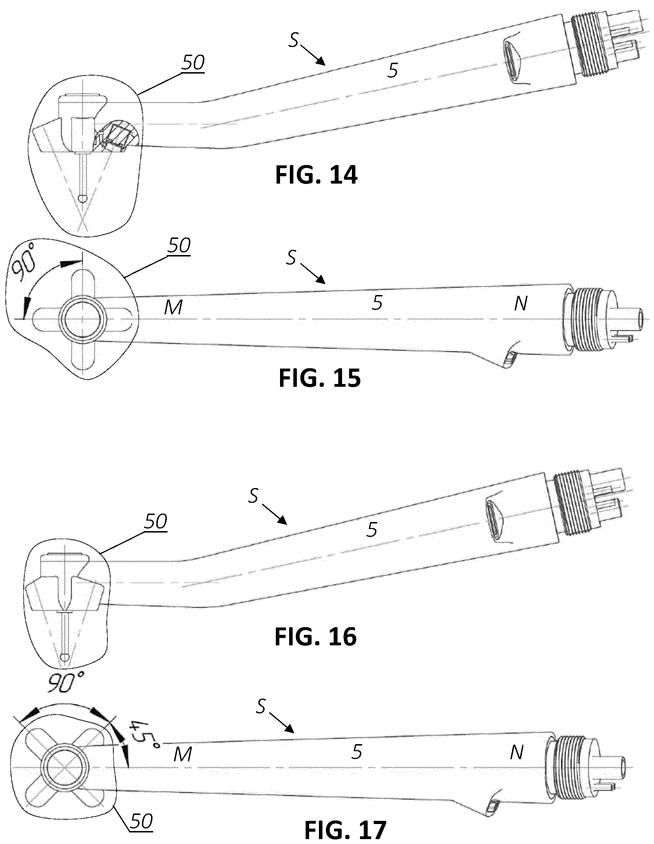

- figures 14 and 15 represent a dental handpiece according to a fifth embodiment of the invention, with four optical sensors demonstrating a position "perpendicularly crosswise, symmetrically relative to the longitudinal axis MN, longitudinally to the longitudinal axis MN" (see figure 15), wherein a first optical sensor is located along the longitudinal axis MN towards the handle 5 (figure 15), a second optical sensor is located on the one side (on the right in the figure 15) from the longitudinal axis MN, perpendicularly to the longitudinal axis MN, a third optical sensor is located along the longitudinal axis MN, in direction from the handle 5 (figure 15), a fourth optical sensor is located on the other side from the longitudinal axis MN (on the left of it in the figure 15, in front of the second optical sensor), perpendicularly to the longitudinal axis MN (figure 15); figure 14 represents a side view of the dental handpiece (with a partial cross-section of the housing 50 elements including the axis MN of the handle 5, figure 15), figure 15 represents a plan view of the dental handpiece;
- figures 16 and 17 represent a dental handpiece according to a sixth embodiment of the invention, with four optical sensors demonstrating a position "perpendicularly crosswise, symmetrically relative to the longitudinal axis MN, at a 45° angle to the longitudinal axis MN" (see figure 17), wherein a first optical sensor is located sidewise of the longitudinal axis MN (on the right of it in the figure 17), along the longitudinal axis MN towards the handle 5 (figure 17), at a 45° angle to said axis MN, a second optical sensor is located sidewise of the longitudinal axis MN (on the right of it in the figure 17), along the longitudinal axis MN, in direction from the handle 5 (figure 17), at a 90° angle to the first optical sensor, a third optical sensor is located sidewise of the longitudinal axis MN (on the left of it in the figure 17), towards the handle 5 (figure 17), at a 45° angle to said axis MN, a fourth optical sensor is located sidewise of the longitudinal axis MN (on the left of it in the figure 17), along the longitudinal axis MN, in direction from the handle 5 (figure 17), at a 90° angle to the third optical sensor; figure 16 represents a side view of the dental handpiece, figure 17 represents a plan view of the dental handpiece.

It is indicated on Figures 1 to 17:
S - Dental handpiece;
MN - preferred axis of the dental handpiece;
1 - Rotor group;
2 - Drill;
3 - Collet clamp button;
4 - Dental handpiece cover;
5 - Dental handpiece handle;
50 - Dental handpiece housing;
6 - Optical sensor sleeve;
7 - Optical sensor board latch;
8 *- "Midwest* 4" adapter;
9 - Optical sensor board protection;
10 - Optical sensor board;
11 - Air supply circuit;
12 - First optical sensor;
120 - Second optical sensor;
13 - First light-emitting diode;
14 - Second diffuser;
15 - First transparent shielding window;
16 - Protrusion;
17 - Connector socket;
18 - Spray supply circuit.

A compressed air source (dental set compressor) and a dental unit are not shown in the figures.

According to figures 1 to 17, a dental handpiece comprises a handle 5 and a housing 50 with an optical emission source represented by at least one first light-emitting diode 13 connected to an electric power supply. Said housing 50 comprises a first optical sensor 12 adapted to be connected to visualization devices.

The housing 50 (more precisely, its optical source) comprises a second light-emitting diode connected to the electric power supply. Said housing 50 comprises a second optical sensor adapted to be connected to the visualization devices. The housing 50 comprises a first transparent shielding window 15 (figures 4 to 5) of the first optical sensor and a second transparent shielding window of the second optical sensor. The handpiece has a means for supplying compressed air on the first and the second shielding windows. The housing 50 comprises a first aperture for supplying compressed air to the first shielding window and a second aperture for supplying compressed air to the second shielding window, both apertures being connected to the means for supplying compressed air. Each aperture has a diffuser that is designed so as to generate an air jet forming an air curtain in front of the transparent shielding window. See figures 1 to 9.

The housing 50 optionally comprises a third light-emitting diode connected to the electric power supply. Said housing 50 (more precisely, its optical source) comprises a third optical sensor adapted to be connected to the visualization devices. See figures 10 to 13.

The housing 50 optionally comprises a fourth light-emitting diode connected to an electric power supply. Said housing 50 (more precisely, its optical source) comprises a fourth optical sensor adapted to be connected to the visualization devices. See figures 14 to 17.

Each diffuser can be located on a side of a working end of the housing and can be designed so as to direct the air jet away from the working end of the housing. See figure 4.

Each diffuser can be located on the handle side of the housing and can be designed so as to direct the airjet away from the handle of the housing. In these conditions, the housing has a protrusion located from the working end, said protrusion having a surface shape which turns the air jet to 120°-140°. See figure 5.

Each diffuser can be oriented perpendicularly to the handle of the housing and can be designed so as to direct the air jet away from the handle of the housing. In these conditions, the housing has a protrusion located from the working end, said protrusion having a surface shape which turns the air jet to 120°-140°.

Each diffuser can be designed so as to generate the air jet with an outflow speed ranging from 6 m/s to 50 m/s and forming an angle from 0°-15° with a plane of the transparent shielding window.

Said dental handpiece is also designed with several air supply options: direct and reverse (with turning of the air curtain), and in different locations of the optical sensors depending on their number from two to four.

The dental handpiece operates as follows. Below is the most exhaustive example of the embodiment of the invention, however, said example does not limit other applications of the invention.

A compressed air is supplied from a dental unit compressor through a compressed air supplying tube 11 as a channeled air jet (inside a handle 5) to a housing 50. When the air passes through the housing 50, it goes to directional diffusers 14 that form a flat jet directed at an angle up to 15 degrees versus a housing surface, with an outflow speed ranging from 6 m/s to 50 m/s. The high-speed jet does not allow foreign particles having densities and speeds typical for dental interventions pass through its core and moves them away from each shielding window of each optical sensor.

Quantitative attributes of the technical parameters such as an air jet turning angle, the airjet outflow speed and the angle between the airjet outflow and the transparent shielding window plane in a range of continuously changing variables can be adjusted by varying a protrusion and the diffuser geometry.

The selection of a specific range of technical parameter values is made experimentally and depends on the efficiency of optical sensor (camera) shielding window blowing. Blowing efficiency control method shall be a visual control of readability of the image transferred from the optical sensor and contaminated with artifacts due to foreign particles and liquid droplets getting onto the shielding window. A number of image artifacts shall be assumed as a criterion of blowing efficiency in four tiers: "none" - there is no visible artifacts from foreign particles and liquid droplets, "moderate" - foreign particles and liquid droplets are not retained on the surface of the transparent shielding window and do not affect a readability of the transferred image, "medium" - foreign particles and liquid droplets remain on the surface of the shielding window and affect the image transferred from the optical sensor, with readability remaining sufficient to direct the dental handpiece in an operating environment, "severe" - foreign particles and liquid droplets remain on the surface of the shielding window, making the image from the optical sensor unreadable and not suitable for correct orientation of the dental handpiece in the operating environment.

An air jet rotation angle is adjusted in the range from 120° to 140° by adjusting a shape of the protrusion surface directed at the diffuser. A value of a protrusion incline angle shall be a geometrical parameter for an air jet turning angle. The protrusion incline angle is found between a tangential line to the margin of the concave surface and a plane of the transparent shielding window. See Table 1 for a relationship between the air jet turning angles, image artifact grade, and the above geometrical parameter. See figure 1 for an example of the embodiment of the invention with borderline values of said range.

**Table 1. Dependence of an air jet turning angle and an image artifact grade from a protrusion incline angle.**

| No | Air jet turning angle, degrees | Value of a protrusion incline angle, degrees | Image artifact grade |
|---|---|---|---|
| 1 | 115 | 85 | Medium |
| 2 | 120 | 80 | Moderate |
| 3 | 125 | 75 | Moderate |
| 4 | 130 | 70 | None |
| 5 | 135 | 65 | None |
| 6 | 140 | 60 | None |

An air jet outflow speed was adjusted in a range from 6 m/s to 50 m/s by adjusting the diffuser outlet cross-section area. A geometrical parameter characterizing the airjet outflow speed was a value of a round section diameter with the same area as the diffuser outlet cross-section. See Table 2 for a relationship between an air jet outflow, an image artifact grade, and the above-mentioned geometrical parameter.

**Table 2. Dependence of air jet outflow speed and image artifact grade from equivalent round section diameter.**

| No | Value of an air jet outflow speed, m/s | Value of an equivalent round section diameter, mm | Image artifact grade |
|---|---|---|---|
| 1 | 3 | 2,8 | Severe |
| 2 | 6 | 2 | Medium |
| 3 | 10 | 1,6 | Moderate |
| 4 | 20 | 1,1 | Moderate |
| 5 | 30 | 0,9 | Moderate |
| 6 | 40 | 0,8 | None |
| 7 | 50 | 0,7 | None |

An angle between an air curtain outflow direction and a shielding window plane was adjusted in a range from 0 to 15 degrees by adjusting a diffuser outlet cross-section aperture angle. The diffuser outlet cross-section aperture angle was assumed as a characteristic geometrical parameter. See Table 3 for a dependence of an angle between the direction of the air curtain outflow direction and shielding window plane, an image artifact grade and the above geometrical parameter.

**Table 3. Dependence of an angle between the direction of the air curtain outflow direction and shielding window plane, an image artifact grade and a diffuser outlet cross-section aperture angle.**

| No | Value of an angle between a direction of the air curtain outflow direction and shielding window plane, degrees | Valure of a diffuser outlet cross-section aperture angle, degrees | Image artifact grade |
|---|---|---|---|
| 1 | 0 | 0 | None |
| 2 | 2,5 | 5 | None |
| 3 | 5 | 10 | Moderate |
| 4 | 7,5 | 15 | Moderate |
| 5 | 10 | 20 | Medium |
| 6 | 12,5 | 25 | Medium |
| 7 | 15 | 30 | Medium |
| 8 | 17,5 | 35 | Severe |

Several optical sensors make it possible to obtain a three-dimensional image of an oral cavity. In other words, an operator of the dental handpiece according to the invention will be able to see a three-dimensional image on a screen during any interventions in the oral cavity.

Furthermore, a virtual three-dimensional (3D) model can be generated using pre-rendered CT images. Said virtual three-dimensional (3D) model can be joined with CT images using optical sensors. Under these conditions, an operator of the dental handpiece according to the invention will be able to view hidden dental cavities and get an understanding how a cutting tip is positioned relative to these cavities.

The claimed dental handpiece may be implemented by a person skilled in the art in practice and ensures that the claimed objectives are met after implementation, which leads to the conclusion that the invention meets the requirement of "industrial applicability".

A series of real-life tests was performed to confirm an operability of the proposed dental handpiece. It has been shown that there is no contamination of the optical windows, and all optical sensors continue to transmit a clear image, which allows for three-dimensional imaging of the oral cavity, throughout a manipulation process.

Therefore, these novel features make it possible to achieve the claimed technical results, namely, to ensure reliable protection of the optical sensors by implementing an air curtain and to obtain a high-quality three-dimensional image of a patient's oral cavity where an operator applies the dental handpiece according to the invention.

Thus, (see figures 1-17), the invention relates to a dental handpiece comprising:
- a handle 5 extending along an axis MN and presenting a first end adapted to be connected to a dental unit and a second free end axially opposite to the first end,
- a housing 50 rigidly connected to the second free end of the handle 5 and comprising:
   ∘ an optical emission source represented by at least one first light-emitting diode 13 connected to an electric power supply,
   ∘ a first optical sensor 12 adapted to be connected to visualization devices.

According to the invention, the optical emission source further comprises a second light-emitting diode different from the first light-emitting diode 13. The second light-emitting diode is connected to the electric power supply. Under these conditions, the housing 50 further comprises:
- a second optical sensor 120 different from the first optical sensor 12, said second optical sensor being adapted to be connected to the visualization devices,
- a first transparent shielding window 15 adapted to protect the first optical sensor 12,
- a second transparent shielding window different from the first transparent shielding window 15 adapted to protect the second optical sensor 120.

Under these conditions, the dental handpiece S further comprise a means for supplying compressed air 11 on the first shielding window 15 and on the second transparent shielding window. The housing 50 further comprises:
- a first aperture adapted for supplying compressed air to the first transparent shielding window 15, said first aperture being connected to the means for supplying compressed air 11,
- a second aperture different from the first aperture, said second aperture being adapted for supplying compressed air to the second transparent shielding window, said second aperture being connected to the means for supplying compressed air 11.

Under these conditions, a first diffuser 14 is located in the first aperture. Said first diffuser 14 is adapted to generate an air jet forming an air curtain in front of the first transparent shielding window 15. A second diffuser different from the first diffuser 14 is located in the second aperture. Said second diffuser is adapted to generate an air jet forming an air curtain in front of the second transparent shielding window.

Preferably, the optical emission source further comprises a third light-emitting diode different from the first light-emitting diode and the second light-emitting diode. Said third light-emitting diode is connected to the electric power supply. The housing 50 further comprises a third optical sensor different from the first optical sensor 12 and the second optical sensor 120. Said third optical sensor is adapted to be connected to the visualization devices.

Preferably, the optical emission source further comprises a fourth light-emitting diode different from the first light-emitting diode, the second light-emitting diode and the third light-emitting diode. Said fourth light-emitting diode is connected to the electric power supply. The housing 50 further comprises a fourth optical sensor different from the first optical sensor 12, the second optical sensor 120 and the third optical sensor. Said fourth optical sensor is adapted to be connected to the visualization devices.

In a first embodiment of the invention (Figure 4), each diffuser among the first diffuser 14 and the second diffuser is located from a working end of the housing 50. Said each diffuser is adapted to direct the air jet away from the working end of the housing 50 along the axis MN of the handle 5.

In an alternative (to the first embodiment above) second embodiment of the invention (Figure 5), each diffuser among the first diffuser 14 and the second diffuser is located from the working end of the housing 50. Said each diffuser is adapted to direct the air jet away from the working end of the housing 50 along the axis MN of the handle 5. The housing 50 comprises a protrusion 16 located from the working end of the housing 50. The protrusion has a surface shape which turns the air jet outgoing from the respective diffuser in a first angle comprising in a first interval from 120° to 140°.

In an alternative (to the first and the second embodiments above) third embodiment of the invention, each diffuser among the first diffuser 14 and the second diffuser is oriented perpendicularly to the handle 5. Each diffuser among the first diffuser 14 and the second diffuser is adapted to direct the air jet sideways from the handle 5. The housing 50 comprises a protrusion located from the working end. The protrusion has a surface shape which turns the air jet outgoing from the respective diffuser by the first angle comprising in the first interval from 120° to 140°.

Preferably, each diffuser among the first diffuser 14 and the second diffuser is adapted to generate the air jet with an outflow speed ranging from 6 m/s to 50 m/s. Said air jet outflow speed forms a second angle with a plane of the transparent shielding window corresponding to said each diffuser, said second angle being comprised in a second interval from 0° to 15°.

Any light-emitting diode among the above first light-emitting diode, second light-emitting diode, third light-emitting diode, fourth light-emitting diode can present punctual geometry. However, preferably, any light-emitting diode from the above has especially a ring pattern as shown in the examples in figures 4 and 5: according to the applicant's observations, such ring pattern ensures the most uniform (without hotspots and/or shadows) lighting of an oral cavity sufficient to obtain a high-quality three-dimensional image.

## Claims

1. A dental handpiece (S), including:
• a handle (5) extending along an axis (MN) and presenting a first end adapted to be connected to a dental unit and a second free end axially opposite to the first end,
• a housing (50) rigidly connected to the second free end of the handle (5) and comprising:
∘ an optical emission source represented by at least one first light-emitting diode (13) connected to an electric power supply,
∘ a first optical sensor (12) adapted to be connected to visualization devices,
**characterized in that** the optical emission source further comprises a second light-emitting diode different from the first light-emitting diode (13),
**in that** the second light-emitting diode is connected to the electric power supply,
**in that** the housing (50) further comprises:
• a second optical sensor (120) different from the first optical sensor (12), said second optical sensor being adapted to be connected to the visualization devices,
• a first transparent shielding window (15) adapted to protect the first optical sensor (12),
• a second transparent shielding window different from the first transparent shielding window (15) adapted to protect the second optical sensor (120),
**in that** the dental handpiece (S) further comprises a means for supplying compressed air (11) on the first shielding window (15) and on the second transparent shielding window,
**in that** the housing (50) additionally comprises:
• a first aperture adapted for supplying compressed air to the first transparent shielding window (15), said first aperture being connected to the means for supplying compressed air (11),
• a second aperture different from the first aperture, said second aperture being adapted for supplying compressed air to the second transparent shielding window, said second aperture being connected to the means for supplying compressed air (11),
**in that** a first diffuser (14) is located in the first aperture,
**in that** said first diffuser (14) is adapted to generate an air jet forming an air curtain in front of the first transparent shielding window (15),
**in that** a second diffuser different from the first diffuser (14) is located in the second aperture,
**in that** said second diffuser is adapted to generate an air jet forming an air curtain in front of the second transparent shielding window.

2. The dental handpiece (S) according to claim 1, **characterized in that** the optical emission source further comprises a third light-emitting diode different from the first light-emitting diode and the second light-emitting diode,
**in that** the third light-emitting diode is connected to the electric power supply,
**in that** the housing (50) further comprises a third optical sensor different from the first optical sensor (12) and the second optical sensor (120), said third optical sensor being adapted to be connected to the visualization devices.

3. The dental handpiece (S) according to claim 1, **characterized in that** the optical emission source further comprises a fourth light-emitting diode different from the first light-emitting diode, the second light-emitting diode and the third light-emitting diode,
**in that** the fourth light-emitting diode is connected to the electric power supply,
**in that** the housing (50) further comprises a fourth optical sensor different from the first optical sensor (12), the second optical sensor (120) and the third optical sensor, said fourth optical sensor being adapted to be connected to the visualization devices.

4. The dental handpiece (S) according to anyone of claims 1 to 3, **characterized in that** each diffuser among the first diffuser (14) and the second diffuser is located from a working end of the housing (50), said each diffuser being adapted to direct the air jet away from the working end of the housing (50) along the axis (MN) of the handle (5).

5. The dental handpiece (S) according to anyone of claims 1 to 3, **characterized in that** each diffuser among the first diffuser (14) and the second diffuser is located from the working end of the housing (50), said each diffuser being adapted to direct the air jet away from the working end of the housing (50) along the axis (MN) of the handle (5),
**in that** the housing (50) comprises a protrusion (16) located from the working end of the housing (50), and
**in that** the protrusion (16) has a surface shape which turns the air jet outgoing from the respective diffuser in a first angle comprising in a first interval from 120° to 140°.

6. The dental handpiece (S) according to anyone of claims 1 to 3, **characterized in that** each diffuser among the first diffuser (14) and the second diffuser is oriented perpendicularly to the handle (5),
**in that** each diffuser among the first diffuser (14) and the second diffuser is adapted to direct the air jet sideways from the handle (5),
**in that** the housing (50) comprises a protrusion located from the working end, and
**in that** the protrusion has a surface shape which turns the air jet outgoing from the respective diffuser by the first angle comprising in the first interval from 120° to 140°.

7. The dental handpiece (S) according to anyone of claims 1 to 3, **characterized in that** each diffuser among the first diffuser (14) and the second diffuser is adapted to generate the air jet with an outflow speed ranging from 6 m/s to 50 m/s, and
**in that** said outflow speed forms a second angle with a plane of the transparent shielding window corresponding to said each diffuser, said second angle being comprised in a second interval from 0° to 15°.
